(19)

**Europäisches Patentamt**

**European Patent Office**

**Office européen des brevets**

(11) **EP 4 775 197 A1**

(12) **EUROPEAN PATENT APPLICATION**

(43) Date of publication:
**15.07.2026 Bulletin 2026/29**

(21) Application number: **25224532.9**

(22) Date of filing: **17.12.2025**

(51) International Patent Classification (IPC):
*A61K 8/04* (2006.01)   *A61K 8/44* (2006.01)
*A61K 8/46* (2006.01)   *A61K 8/60* (2006.01)
*A61Q 5/02* (2006.01)   *A61K 8/49* (2006.01)
*A61K 8/81* (2006.01)   *A61K 8/86* (2006.01)

(52) Cooperative Patent Classification (CPC):
**A61K 8/466; A61K 8/042; A61K 8/44; A61K 8/442;
A61K 8/4946; A61K 8/604; A61K 8/8147;
A61K 8/86; A61Q 5/02;** A61K 2800/596

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority:  **06.01.2025 JP 2025001965**

(71) Applicant: **Shiseido Honeycake Industries Co.,
Ltd.
Ibaraki-shi, Osaka 567-0021 (JP)**

(72) Inventors:
• **NISHINA, Tetsuo**
  **Osaka, 567-0021 (JP)**
• **HARADA, Koki**
  **Osaka, 567-0021 (JP)**

(74) Representative: **Henkel & Partner mbB
Patentanwaltskanzlei, Rechtsanwaltskanzlei
Maximiliansplatz 21
80333 München (DE)**

(54) **GEL HAIR CLEANSING AGENT**

(57) A gel hair cleansing agent comprising: (A) acyl methyl taurate and acyl methyl alaninate; (B) at least one selected from alkylamidopropyl betaine and imidazolinium betaine; (C) 0.5% by mass to 3% by mas s of polyoxyethylene methyl glucoside dioleate; and (D) at least one selected from an acrylic polymer and a reaction product of adipic acid and acetic anhydride and corn starch, wherein the total content of the component (A) and the component (B) is 10% by mass to 25% by mass, a mass ratio of the component (A) and the component (B) [(A)/(B)] is 0.3 to 0.9, and viscosity at 25C° is 6,000 mPa·s or greater is provided.

EP 4 775 197 A1

**Description**

Technical Field

The present invention relates to a gel hair cleansing agent.

Background Art

Shampoo generally comprises a sulfuric-acid based anionic surfactant such as sodium lauryl ether sulphate (LES) and an amphoteric surfactant such as cocamidopropyl betaine as a cleansing component, and comprises table salt as a viscosity modifier.

In recent years, an amino-acid based surfactant such as N-acyl-N-alkylglycine salt is used instead of LES; however, since thickening is difficult with an amino-acid based surfactant, a viscosity modifier such as an acrylic polymer, for example, is added to achieve texture and high viscosity like a gel.

Moreover, a skin cleansing composition comprising polyoxyethylene methyl glucoside dioleate as a thickener is proposed (for example, refer to Japanese Patent No. 6181309 B).

Summary of Invention

Technical Problem

As described above, a characteristic texture and high viscosity can be imparted by adding a viscosity modifier such as an acrylic polymer to an amino-acid based surfactant; however, there is a problem that squeakiness occurs to hair at use.

Moreover, in the above-described Japanese Patent No. 6181309 B, the content of polyoxyethylene methyl glucoside dioleate is small, such as 0.02% by mass to 0.1% by mass, so that it is difficult to impart texture and high viscosity like a gel.

The object of the present invention is to solve the above-described conventional problems, and achieve the following objects. That is, the object of the present invention is to provide a gel hair cleansing agent that can achieve a highly viscous gel, is excellent in foaming property and usability, and has a good storage stability at high temperature (approximately 50C°).

Solution to Problem

The present inventors carried out intensive studies to achieve the above-described objects, and as a result, they found that a gel hair cleansing agent comprising: (A) acyl methyl taurate and acyl methyl alaninate; (B) at least one selected from alkylamidopropyl betaine and imidazolinium betaine; (C) polyoxyethylene methyl glucoside dioleate; and (D) at least one selected from an acrylic polymer and a reaction product of adipic acid and acetic anhydride, and corn starch, and the total content of the component (A) and the component (B) is 10% by mass to 25% by mass, and a mass ratio of the component (A) and the component (B) [(A)/(B)] is 0.3 to 0.9 can achieve a highly viscous gel, is excellent in foaming property and usability, and has a good storage stability at high temperature, and thus completed the present invention.

The present invention is based on the above-described findings by the present inventors, and means to solve the above-described problems is as follows.

A gel hair cleansing agent of the present invention comprises: (A) acyl methyl taurate and acyl methyl alaninate; (B) at least one selected from alkylamidopropyl betaine and imidazolinium betaine; (C) 0.5% by mass to 3% by mass of polyoxyethylene methyl glucoside dioleate; and (D) at least one selected from an acrylic polymer and a reaction product of adipic acid and acetic anhydride and corn starch, wherein the total content of the component (A) and the component (B) is 10% by mass to 25% by mass, a mass ratio of the component (A) and the component (B) [(A)/(B)] is 0.3 to 0.9, and viscosity at 25C° is 6,000 mPa·s or greater.

Moreover, in the gel hair cleansing agent, the content of the component (A) is preferably 1 % by mass to 15 % by mass.

Moreover, in the gel hair cleansing agent, a mass ratio of acyl methyl taurate (A1) and acyl methyl alaninate (A2) [(A1)/(A2)] is preferably 0.2 to 2.

Moreover, in the gel hair cleansing agent, the component (B) preferably comprises at least one selected from cocamidopropyl betaine and sodium lauroamphoacetate.

Moreover, in the gel hair cleansing agent, the component (C) preferably comprises PEG-120 methyl glucose dioleate.

Moreover, in the gel hair cleansing agent, the component (D) preferably comprises at least one selected from acrylates copolymer and starch acetate/adipate.

Moreover, in the gel hair cleansing agent, the content of the component (D) is preferably 0.1% by mass to 1.2% by mass.

Moreover, the gel hair cleansing agent preferably comprises (E) cationic polymer.

Moreover, in the gel hair cleansing agent, the pH is preferably 7 or lower.

Moreover, the gel hair cleansing agent is preferably used in shampoo or an all-in-one shampoo.

Advantageous Effects of Invention

According to the present invention, a gel hair cleansing agent that solves the above-described conventional problems, can achieve the above-described objects, can achieve a highly viscous gel, is excellent in foaming property and usability, and has a good storage stability at high temperature can be provided.

Description of Embodiments

A gel hair cleansing agent of the present invention comprises: (A) acyl methyl taurate and acyl methyl alaninate; (B) at least one selected from alkylamidopropyl betaine and imidazolinium betaine; (C) polyoxyethylene methyl glucoside dioleate; and (D) at least one selected from an acrylic polymer and a reaction product of adipic acid and acetic anhydride and corn starch, and further comprises other components as necessary.

In the present invention, a "gel (gel-state or gel-type)" means a semi-solid state having fluidity, and viscosity at 25C° is 6,000 mPa·s or greater, preferably 8,000 mPa·s or greater, more preferably 10,000 mPa·s or greater, and further preferably 15,000 mPa·s or greater. The upper limit of viscosity at 25C° is preferably 30,000 mPa·s or lower in terms of handleability.

Viscosity complies with a viscosity measuring method described in the general test of Japanese Pharmacopoeia 17th Edition, and is a value measured by a B-type viscometer at 25C°.

<Acyl methyl taurate and Acyl methyl alaninate>

- (A1) Acyl methyl taurate -

Acyl methyl taurate of the component (A) is an anionic surfactant, and is a salt that combined an acyl group and a methyl group to an amino group of taurine (2-aminoethyl-1-sulfonic acid).

An acyl group is not particularly limited, and is preferably a fatty acid residue having 8 to 22 carbon atoms. Examples thereof include: single acyl groups such as lauric acid, myristic acid, palmitic acid, stearic acid, isostearic acid, and oleic acid; acyl groups deriving from coconut oil fatty acid, beef tallow fatty acid, castor oil fatty acid, olive oil fatty acid, and palm oil fatty acid, and combinations thereof.

Examples of a salt of acyl methyl alaninate include: alkaline metal salts such as sodium salt and potassium salt; amine salts such as monoethanolamine salt, diethanolamine salt, and triethanolamine salt; and triethanolamine salt. Among them, sodium salt and potassium salt are preferred.

Examples of acyl methyl taurate of the component (A1) include sodium methyl lauroyl taurate, sodium methyl myristoyl taurate, sodium methyl palmitoyl taurate, sodium methyl stearoyl taurate, sodium methyl tallow fatty acid taurate, sodium methyl coconut oil fatty acid taurate (sodium methyl cocoyl taurate), and sodium methyl decanoyl taurate. They may be used alone, or in combination of two or more types. Among them, sodium methyl cocoyl taurate is preferred.

- (A2) Acyl methyl alaninate -

Acyl methyl alaninate of the component (A2) is an anionic surfactant, and is a salt of amides of an acyl group and methyl alanine.

An acyl group is not particularly limited, and is preferably a fatty acid residue having 8 to 22 carbon atoms. Examples thereof include: single acyl groups such as lauric acid, myristic acid, palmitic acid, stearic acid, isostearic acid, and oleic acid; acyl groups deriving from coconut oil fatty acid, beef tallow fatty acid, castor oil fatty acid, olive oil fatty acid, and palm oil fatty acid, and combinations thereof.

Examples of a salt of acyl methyl alaninate include: alkaline metal salts such as sodium salt and potassium salt; amine salts such as monoethanolamine salt, diethanolamine salt, and triethanolamine salt; and triethanolamine salt. Among them, sodium salt and potassium salt are preferred.

Examples of acyl methyl taurate of the component (A2) include sodium lauroyl methylaminopropionate, triethanolamine lauroyl methylaminopropionate, sodium myristoyl methylaminopropionate, and sodium cocoyl methylaminopropionate (sodium cocoyl methyl beta-alanine). They may be used alone, or in combination of two or more types.

Among them, sodium lauroyl methylaminopropionate is preferred.

The total content of acyl methyl taurate and acyl methyl alaninate of the component (A) is preferably 1% by mass to 15% by mass, more preferably 1% by mass to 10% by mass, and further preferably 3% by mass to 10% by mass relative to the total amount of the gel hair cleansing agent.

The mass ratio of acyl methyl taurate (A1) and acyl methyl alaninate (A2) [(A1)/(A2)] is preferably 0.2 to 2, and more preferably 0.2 to 1. When the mass ratio [(A1)/(A2)] is 0.2 to 2, a high viscosity range of the gel hair cleansing agent can be achieved.

<(B) At least one selected from alkylamidopropyl betaine and imidazolinium betaine>

At least one selected from alkylamidopropyl betaine and imidazolinium betaine may be used alone or in combination of two or more types.

- (B1) Alkylamidopropyl betaine -

Alkylamidopropyl betaine of the component (B1) is an amphoteric surfactant, and examples thereof include coconut oil fatty acid amidopropyl betaine (cocamidopropyl betaine), palm kernel oil fatty acid amidopropyl betaine, lauramidopropyl betaine, myristic acid amidopropyl betaine (myristamidopropyl betaine), cocamidopropyl betaine, and undecylenamidopropyl betaine. They may be used alone, or in combination of two or more types. Among them, coconut oil fatty acid amidopropyl betaine (cocamidopropyl betaine) is preferred.

- (B2) Imidazolinium betaine -

Imidazolinium betaine of the component (B2) is an amphoteric surfactant, and examples thereof include 2-alkyl-N-carboxymethyl-N-hydroxyethylimidazolinium betaine (sodium cocoamphoacetate), sodium N-lauroyl-N'-carboxymethyl-N'-hydoxyethyl ethylenediamine (sodium lauroamphoacetate), cocoyl alkyl-N-hydroxyethyl imidazolinium betaine, sodium 2-undecyl-N,N,N-(hydroxyethylcarboxylmethyl)-2-imidazoline, 2-cocoyl-2-imidazolinium hydroxide-1-carboxyethyloxy disodium salt, and 2-heptadecyl-N-carboxymethyl-N-hydroxyethyl imidazolinium betaine. They may be used alone, or in combination of two or more types. Among them, sodium N-lauroyl-N'-carboxymethyl-N'-hydoxyethyl ethylenediamine (sodium lauroamphoacetate) is preferred.

The content of at least one selected from alkylamidopropyl betaine and imidazolinium betaine of the component (B) is preferably 1% by mass to 15% by mass, more preferably 1% by mass to 10% by mass, and further preferably 3% by mass to 10% by mass relative to the total amount of the gel hair cleansing agent.

The total content of the component (A) and the component (B) is 10% by mass to 25% by mass, preferably 10% by mass to 20% by mass, and more preferably 15% by mass to 20% by mass. When the total content of the component (A) and the component (B) is 10% by mass to 25% by mass, foaming property improves, and a high viscosity range of the gel hair cleansing agent can be achieved.

The mass ratio of the component (A) and the component (B) [(A)/(B)] is 0.3 to 0.9, preferably 0.3 to 0.7, and more preferably 0.5 to 0.7. When the mass ratio [(A)/(B)] is 0.3 to 0.9, a high viscosity range of the gel hair cleansing agent can be achieved.

<(C) Polyoxyethylene methyl glucoside dioleate>

Polyoxyethylene methyl glucoside dioleate of the component (C) is polyethylene glycol ether consisting of a diester of oleic acid and methyl glucose.

Examples of polyoxyethylene methyl glucoside dioleate of the component (C) include PEG-120 methyl glucose dioleate. PEG-120 methyl glucose dioleate is a labeling name defined by Japan Cosmetic Industry Association.

The content of polyoxyethylene methyl glucoside dioleate of the component (C) is 0.5% by mass to 3% by mass, preferably 1% by mass to 3% by mass, and more preferably 1% by mass to 2% by mass relative to the total amount of the gel hair cleansing agent. When the content of polyoxyethylene methyl glucoside is 0.5 % by mass to 3% by mass, an excellent effect of increasing viscosity can be achieved.

<(D) At least one selected from an acrylic polymer and a reaction product of adipic acid and acetic anhydride and corn starch>

At least one selected from an acrylic polymer and a reaction product of adipic acid and acetic anhydride and corn starch of the component (D) may be used alone, or in combination of two or more types.

In the gel hair cleansing agent of the present invention, polyoxyethylene methyl glucoside of the component (C) and at least one selected from an acrylic polymer and a reaction product of acetic anhydride and corn starch of the component (D) are incorporated together, so that storage stability at high temperature improves, and an excellent effect of increasing viscosity can be achieved at the same time.

An acrylic polymer is preferably a copolymer of two or more types of constituent monomers selected from a group consisting of acrylic acid, methacrylic acid, alkyl (C1-C2) acrylate, and alkyl (C1-C4) methacrylate.

In the present invention, an acrylic copolymer or a methacrylic copolymer that is a random copolymer, an alternating copolymer, or a block copolymer can be used.

Examples of an acrylic polymer include compounds represented with INCI name (International Cosmetic Ingredient Dictionary and Handbook, 14th Edition, Volume 1, CTFA, 2012, p. 60-61) of "ACRYLATES COPOLYMER".

Examples of the reaction product of adipic acid and acetic anhydride and corn starch include starch acetate/adipate (cosmetic labeling name).

The content of at least one selected from an acrylic polymer and a reaction product of adipic acid and acetic anhydride and corn starch of the component (D) is preferably 0.1% by mass to 1.2% by mass, more preferably 0.2% by mass to 1.0% by mass, and further preferably 0.3% by mass to 0.9% by mass relative to the total amount of the gel hair cleansing agent. When the content of the component (D) is 0.1% by mass to 1.2% by mass, storage stability at high temperature improves, and an excellent effect of increasing viscosity can be achieved at the same time.

Other than the above-described components (A) to (D), it is preferred that the gel hair cleansing agent of the present invention further comprises (E) a cationic polymer, and (F) a silicone emulsion, as necessary.

<(E) Cationic polymer>

Examples of the cationic polymer of the component (E) include O-[2-hydroxy-3-(trimethylammonio)propyl] hydroxyethylcellulose chloride (cationic cellulose; Polyquaternium-10), O-[2-hydroxy-3-(trimethylammonio)propyl] guar gum chloride (cationic guar gum; guar hydroxypropyltrimonium chloride), dimethyldiallyl ammonium chloride/acrylamide copolymer (Polyquaternium-7), cationic tamarind gum, cationic fenugreek gum, cationic tara gum, cationic locust bean gum, vinylpyrrolidone/quaternized dimethylaminoethyl methacrylate copolymer, and polyvinylpyrrolidone/alkylamino acrylate copolymer. They may be used alone, or in combination of two or more types.

The content of the cationic polymer of the component (E) is preferably 0.1% by mass to 10% by mass, and more preferably 0.3% by mass to 5% by mass relative to the total amount of the gel hair cleansing agent.

<(F) Silicone emulsion>

A silicone emulsion of the component (F) means a water-emulsified silicone emulsion in which silicones are emulsified with water by using a surfactant. The content of silicone contained in this silicone emulsion is not particularly limited; however, it is preferably 35% by mass to 60% by mass in the emulsion, in terms of availability.

Examples of the silicone emulsion include: those that emulsified dimethyl polysiloxane with a surfactant; those that mixed a high-viscosity dimethyl polysiloxane with a low-viscosity dimethyl polysiloxane, decamethylcyclopentasiloxane or light liquid paraffin, and emulsified the mixture with water; those that emulsified a highly-polymerized methyl polysiloxane and a low-viscosity methyl polysiloxane with a surfactant; and those that emulsified a highly-polymerized methyl polysiloxane and a low-viscosity methyl polysiloxane with a surfactant under the presence of a polyhydric alcohol.

The content of the silicone emulsion of the component (F) is preferably 0.1% by mass to 5% by mass, and more preferably 0.1% by mass to 3% by mass relative to the total amount of the gel hair cleansing agent.

<Other components>

The gel hair cleansing agent of the present invention can comprise other components within the range in which the effects of the present invention are not impaired.

Examples of other components include water, cationic surfactants, nonionic surfactants, moisturizers, water-soluble polymers, coating agents, UV absorbers, sequestering agents, lower alcohols, polyhydric alcohols, oil components, sugars, amino acids, organic amines, polymer emulsions, pH adjusters, skin nutrients, vitamins, antioxidants, antioxidant aids, hair-growth components, fragrances, colorants, and preservatives, which are appropriately incorporated as necessary, thereby producing a gel hair cleaning agent according to the desired dosage form.

Water that is used in cosmetics and quasi-drugs can be used, and examples thereof include ion-exchanged water, ultrafiltered water, reverse osmosis water, distilled water, purified water, pure water, ultrapure water, and tap water.

The water content is preferably 10 % by mass to 80% by mass relative to the total amount of the gel hair cleansing agent, more preferably 20 % by mass to 70% by mass, even more preferably 30 % by mass to 70% by mass, and particularly preferably 40 % by mass to 70% by mass.

Examples of cationic surfactants include alkyltrimethylammonium salts (e.g., stearyltrimethylammonium chloride and behenyltrimethylammonium chloride); alkylpyridinium salts (e.g., cetylpyridinium chloride); distearyldimethylammonium chloride and dialkyldimethylammonium salts; poly(N,N'-dimethyl-3,5-methylene piperidinium) chloride; alkyl quaternary ammonium salts; alkyldimethylbenzylammonium salts; alkylisoquinolinium salts; dialkylmorphonium salts; POE-alkyl amines; alkyl amine salts; polyamine fatty acid derivatives; amyl alcohol fatty acid derivatives; benzalkonium chloride; benzethonium chloride; and the like.

Examples of lipophilic nonionic surfactants include sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate); glycerin polyglycerin fatty acids (e.g., mono cottonseed oil fatty acid glycerin, glycerin monoerucate, isopentyl diol, glycerin sesquioleate, glycerin monostearate, glycerin $\alpha,\alpha'$-oleate pyroglutamate, and glyceryl monostearate); propylene glycol fatty acid esters (e.g., propylene glycol monostearate); hydrogenated castor oil derivatives; glycerin alkyl ethers, and the like.

Examples of hydrophilic nonionic surfactants include POE-sorbitan fatty acid esters (e.g., POE-sorbitan monooleate, POE-sorbitan monostearate, and POE-sorbitan tetraoleate); POE-sorbitol fatty acid esters (e.g., POE-sorbitol monolaurate, POE-sorbitol monooleate, POE-sorbitol pentaoleate, and POE-sorbitol monostearate); POE-glycerin fatty acid esters (e.g., POE-monooleates, such as POE-glycerin monostearate, POE-glycerin monoisostearate, and POE-glycerin triisostearate); POE-fatty acid esters (e.g., POE-distearate, POE-monodioleate, and ethylene glycol distearate); POE-alkyl ethers (e.g., POE-lauryl ether, POE-oleyl ether, POE-stearyl ether, POE-behenyl ether, POE-2-octyldodecyl ether, and POE-cholestanol ether); Pluronic (registered trademark) series; POE/POP-alkyl ethers (e.g., POE/POP-cetyl ether, POE/POP-2-decyltetradecyl ether, POE/POP-monobutyl ether, POE/POP-hydrogenated lanolin, and POE/POP-glycerin ether); tetra-POE/tetra-POP-ethylenediamine condensates (e.g., Tetronic); POE-castor oil hydrogenated castor oil derivatives (e.g., POE-castor oil, POE-hydrogenated castor oil, POE-hydrogenated castor oil monoisostearate, POE-hydrogenated castor oil triisostearate, POE-hydrogenated castor oil monopyroglutamate monoisostearate diester, and POE-hydrogenated castor oil maleate); POE-beeswax lanolin derivatives (e.g., POE-sorbitol beeswax); alkanolamides (e.g., coconut oil fatty acid diethanolamide, lauric acid monoethanolamide, fatty acid isopropanolamide, and cocamide methyl monoethanolamide); POE-propylene glycol fatty acid esters; POE-alkylamines; POE-fatty acid amides; diethylene glycol laurate; sucrose fatty acid esters; alkylethoxydimethylamine oxides; trioleyl phosphate; and the like.

Examples of moisturizers include polyethylene glycol, propylene glycol, dipropylene glycol, glycerin, 1,3-butylene glycol, xylitol, sorbitol, maltitol, chondroitin sulfate, hyaluronic acid, mucoitin sulfate, caronic acid, atelocollagen, cholesteryl-12-hydroxystearate, sodium lactate, bile salts, dl-pyrrolidone carboxylate, alkylene oxide derivatives, short-chain soluble collagen, diglycerin (EO) PO adducts, Rosa roxburghii extract, yarrow extract, melilot extract, and the like.

Examples of natural water-soluble polymers include plant-based polymers (e.g., gum arabic, tragacanth gum, galactan, guar gum, carob gum, karaya gum, carrageenan, pectin, agar, quince seed (marmelo), algae colloid (brown alga extract), starches (e.g., rice, corn, potato, and wheat), and glycyrrhizic acid); microorganism-based polymers (e.g., xanthan gum, dextran, succinoglycan, and pullulan); animal-based polymers (e.g., collagen, casein, albumin, and gelatin); and the like.

Examples of semi-synthetic water-soluble polymers include starch-based polymers (e.g., carboxymethyl starch and methylhydroxypropyl starch); cellulose-based polymers (methylcellulose, ethylcellulose, methylhydroxypropylcellulose, hydroxyethylcellulose, sodium cellulose sulfate, hydroxypropylcellulose, carboxymethylcellulose, carboxymethylcellulose sodium, crystalline cellulose, and cellulose powder); alginic acid-based polymers (e.g., sodium alginate and alginate propylene glycol ester); and the like.

Examples of synthetic water-soluble polymers include vinyl-based polymers (e.g., polyvinyl alcohol, polyvinyl methyl ether, polyvinylpyrrolidone, and carboxyvinyl polymer); polyoxyethylene-based polymers (e.g., polyoxyethylene polyoxypropylene copolymers of polyethylene glycol 20,000, 40,000, or 60,0000, and highly polymerized polyethylene glycol); acrylic polymers (e.g., sodium polyacrylate, polyethyl acrylate, and polyacrylamide); polyethyleneimine; cationic polymers, and the like.

Examples of UV absorbers include benzoic acid-based UV absorbers (e.g., paraaminobenzoic acid (hereinafter abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, and N,N-dimethyl PABA ethyl ester); anthranilic acid-based UV absorbers (e.g., homomenthyl-N-acetylanthranilate); salicylic acid-based UV absorbers (e.g., amyl salicylate, menthyl salicylate, homomenthyl salicylate, octyl salicylate, phenyl salicylate, benzyl salicylate, and p-isopropanol phenylsalicylate); cinnamic acid-based UV absorbers (e.g., octylmethoxycinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, octyl-p-methoxycinnamate(2-ethylhexyl-p-methoxycinnamate), 2-ethoxyethyl-p-methoxycinnamate, cyclohexyl-p-methoxycinnamate, ethyl-$\alpha$-cyano-$\beta$-phenylcinnamate, 2-ethylhexyl-$\alpha$-cyano-$\beta$-phenylcinnamate, and glyceryl mono-2-ethylhexanoyl-diparamethoxycinnamate); benzophenone-based UV absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, and 4-hydroxy-3-carboxybenzophenone); 3-(4'-methylbenzylidene)-d,l-camphor, 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenylbenzotriazole; dibenzalazine; dianisoylmethane; 4-methoxy-4'-t-butyldibenzoylmethane; 5-(3,3-dimethyl-2-norbornylidene)-3-pentan-2-one, dimorpholinopyridazinone; 2-ethylhexyl-2-cyano-3,3-diphenylacrylate; 2,4-bis-{[4-(2-ethylhexyloxy)-2-hydroxy]-phenyl}-6-(4-methoxyphenyl)-(1,3,5)-triazine; and the like.

Examples of sequestering agents include 1-hydroxyethane-1,1-diphosphonic acid, 1-hydroxyethane-1,1-diphosphonic acid tetrasodium salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium ethylenediaminehydroxyethyl triacetate, and the like.

Examples of lower alcohols include ethanol, propanol, isopropanol, isobutyl alcohol, t-butyl alcohol, and the like.

Examples of polyhydric alcohols may include dihydric alcohol (e.g., ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol); trihydric alcohol (e.g., glycerin, trimethylolpropane); tetrahydric alcohol (e.g., pentaerythritol such as 1,2,6-hexanetriol); pentahydric alcohol (e.g., xylitol); hexahydric alcohol (e.g., sorbitol, mannitol); polyhydric alcohol polymers (e.g., diethylene glycol, dipropylene glycol, triethylene glycol, polypropylene glycol, tetraethylene glycol, diglycerin, polyethylene glycol, triglycerin, tetraglycerin, polyglycerin); dihydric alcohol alkyl ethers (e.g., ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether); dihydric alcohol alkyl ethers (e.g., diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether); dihydric alcohol ether ethers (e.g., ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate); glycerin monoalkyl ethers (e.g., chimyl alcohol, selachyl alcohol, batyl alcohol); sugar alcohol (e.g., sorbitol, maltitol, mannitol, erythritol, xylitol, starch sugar hydrogenated alcohol); glysolid, tetrahydrofurfuryl alcohol; POE-tetrahydrofurfuryl alcohol; POP-butyl ether; POP/POE-butyl ether; tripolyoxypropylene glycerin ether; POP-glycerin ether; POP-glycerin ether phosphoric acid; POP/POE-pentaerythritol ether; polyglycerin, and the like.

Examples of monosaccharides include trioses (e.g., D-glyceryl aldehyde and dihydroxyacetone); tetroses (e.g., D-erythrose, D-erythrulose, D-threose, and erythritol); pentoses (e.g., L-arabinose, D-xylose, L-lyxose, D-arabinose, D-ribose, D-ribulose, D-xylulose, and L-xylulose); hexoses (e.g., D-glucose, D-talose, D-psicose, D-galactose, D-fructose, L-galactose, L-mannose, and D-tagatose); heptoses (e.g., aldoheptose and heptose); octoses (e.g., octulose); deoxy sugars (e.g., 2-deoxy-D-ribose, 6-deoxy-L-galactose, and 6-deoxy-L-mannose); amino sugars (e.g., D-glucosamine, D-galactosamine, sialic acid, aminouronic acid, and muramic acid); uronic acids (e.g., D-glucuronic acid, D-mannuronic acid, L-guluronic acid, D-galacturonic acid, and L-iduronic acid); and the like.

Examples of oligosaccharides include sucrose, gentianose, umbelliferose, lactose, planteose, isolychnose, $\alpha,\alpha$-trehalose, raffinose, lychnose, umbilicin, stachyose verbascose, and the like.

Examples of polysaccharides include cellulose, quince seed, chondroitin sulfate, starch, galactan, dermatan sulfate, glycogen, gum arabic, heparan sulfate, hyaluronic acid, tragacanth gum, keratan sulfate, chondroitin, mucoitin sulfate, dextran, keratosulfate, locust bean gum, succinoglycan, caronic acid, and the like.

Examples of amino acids include neutral amino acids (e.g., threonine and cysteine); basic amino acids (e.g., hydroxylysine); and the like. Examples of amino acid derivatives include acyl sarcosine sodium (e.g., sodium lauroyl sarcosine), acyl glutamate, sodium acyl $\beta$-alanine, glutathione, pyrrolidonecarboxylic acid, and the like.

Examples of organic amines include monoethanolamine, diethanolamine, triethanolamine, morpholine, triisopropanolamine, 2-amino-2-methyl-1,3-propanediol, 2-amino-2-methyl-1-propanol, and the like.

Examples of polymer emulsions include acrylic resin emulsion, polyacrylic acid ethyl emulsion, acrylic resin liquid, polyacrylic alkyl ester emulsion, polyvinyl acetate resin emulsion, natural rubber latex, and the like.

Examples of pH adjusters include buffers such as citric acid, lactic acid-sodium lactate, citric acid-sodium citrate, and succinic acid-sodium succinate.

Examples of vitamins include vitamins A, $B_1$, $B_2$, $B_6$, C, and E, or derivatives thereof, pantothenic acid or derivatives thereof, biotin, and the like.

7

Examples of antioxidants include tocopherols, dibutylhydroxytoluene, butylhydroxyanisole, gallic acid esters, and the like.

Examples of antioxidant aids include phosphoric acid, citric acid, ascorbic acid, maleic acid, malonic acid, succinic acid, fumaric acid, cephalin, hexametaphosphate, phytic acid, ethylenediaminetetraacetic acid, and the like.

Examples of hair-growth components include procyanidin, dipotassium glycyrrhizate, carpronium chloride, menthol, hinokitiol, L-hydroxyproline, acetyl hydroxyproline, fucoidan, swertiamarin, panax ginseng, flavonosteroid, FGF-10, Isodonis japonicus extract, Swertia herb extract, ribbon weed extract, five-leaf ginseng extract, St. John's wort extract, gentian extract, sage extract, peppermint extract, hop extract, persimmon leaf extract, rehmannia root extract, carrot extract, Bohdi tree extract, moutan bark extract, and the like.

Examples of other components that can be incorporated include preservatives (e.g., ethylparaben, butylparaben, chlorphenesin, and phenoxyethano); disinfectants (e.g., isopropyl methylphenol, benzalkonium chloride, benzethonium chloride, and chlorhexidine gluconate); antiphlogistics (e.g., glycyrrhizic acid derivatives, glycyrrhetinic acid derivatives, salicylic acid derivatives, hinokitiol, zinc oxide, and allantoin); various extracts (e.g., phellodendron bark, Coptis japonica, lithospermum root, peony root, Swertia japonica, birch, sage, loquat, carrot, aloe, mallow, iris, grape, coix seed, loofah, lily, saffron, cnidium rhizome, ginger, hypericum, ononis, garlic, chili pepper, chenpi, Japanese angelica root, and seaweed), activators (e.g., royal jelly, photosensitizers, and cholesterol derivatives); blood circulation enhancers (e.g., vanillylamide nonylate, nicotinic acid benzyl ester, nicotinic acid β-butoxyethyl ester, capsaicin, zingerone, cantharis tincture, ichthammol, tannic acid, α-borneol, tocopherol nicotinate, inositol hexanicotinate, cyclandelate, cinnarizine, tolazoline, acetylcholine, verapamil, cepharanthine, and γ-oryzanol); antiseborrheric agents (e.g., sulfur and thianthol); antiinflammatory agents (e.g., tranexamic acid, thiotaurine, and hypotaurine); sequestering agents such as disodium edetate, trisodium edetate, sodium citrate, sodium polyphosphate, sodium metaphosphate, gluconic acid, and malic acid; caffeine, tannin, verapamil, tranexamic acid or derivatives thereof; various crude drug extracts such as licorice, quince, and shinleaf; drugs such as tocopherol acetate, glycyrrhetinic acid, glycyrrhizic acid and derivatives thereof; and the like.

The pH of the gel hair cleansing agent of the present invention is preferably 7 or lower, more preferably 2.5 to 7, and further preferably 3 to 6. The gel hair cleansing agent having the pH of 7 or lower has a good usability (smoothness of combing hair with fingers and combing hair with a comb).

The pH of the gel hair cleansing agent can be measured with a method defined in the general test methods of the Japanese standards of cosmetics ingredients (2nd edition), by inserting an electrode of a pH meter directly into the gel hair cleansing agent at 25°C, and reading a pH value after it is stable.

The gel hair cleansing agent of the present invention can be used preferably in shampoo, all-in-one shampoo, and two-in-one shampoo.

Examples

Examples of the present invention are described in the following; however, the present invention is not limited to these examples in any way. The contents of each component described in the test examples are in "% by mass", and are values in terms of pure content.

The evaluation methods and the measurement methods used in the present invention are as follows.

<pH>

The pH of the gel hair cleansing agent was measured with a method defined in the general test methods of the Japanese standards of cosmetics ingredients (2nd edition), by inserting an electrode of a pH meter (manufactured by DKK-TOA CORPORATION) directly into the gel hair cleansing agent at 25°C, and reading a pH value after it is stable.

<Viscosity>

The viscosity of the gel hair cleansing agent was measured with a B-type viscometer (Brookfield viscometer, manufactured by Brookfield Corporation) at 25°C in accordance with the viscosity measurement method described in the general test methods of Japanese Pharmacopoeia 17th Edition.

<Usability>

- Evaluation on smoothness of combing hair with fingers and combing hair with a comb -

Hair was wetted with warm water at 40°C, and the prepared gel hair cleansing agent was spread and applied directly on hair with hands. After foaming and washing, five expert evaluation panelists performed sensory evaluation on smoothness of combing hair with fingers and combing hair with a comb before and after the gel hair cleansing agent is washed off with warm water at 40°C (during and after use).

[Evaluation criteria]

A: No problem (smoothness of combing with fingers and combing hair with a comb is good)
B: Slightly problematic (smoothness of combing with fingers and combing hair with a comb is slightly bad)
C: Problematic (smoothness of combing with fingers and combing hair with a comb is bad)

<Foaming property>

After stirring 400 mL of a 2% by mass aqueous solution of the prepared gel hair cleansing agent for one minute at a maximum stirring rate of 4,300 rpm at 25°C, the amount of liquid (mL) was measured, and foaming property was evaluated based on the following criteria. In the present invention, "B" or greater is regarded as an acceptable level.

[Evaluation criteria]

A: The amount of liquid is 2,000 mL or greater
B: The amount of liquid is 1,500 mL or greater and less than 2,000 mL
C: The amount of liquid is 1,000 mL or greater and less than 1,500 mL
D: The amount of liquid is less than 1,000 mL

<Storage stability at high temperature>

95 mL of the prepared gel hair cleansing agent was put into a 100 mL sealed container with a lid, and stored in a thermostatic chamber for four weeks at 50°C. The viscosity before and after storage at 25°C was measured with a B-type viscometer (Brookfield viscometer, manufactured by Brookfield Corporation). The change rate of the viscosity was calculated from the following formula 1, and storage stability at high temperature was evaluated based on the following criteria. In the present invention, "B" or greater is regarded as an acceptable level.

Change rate of viscosity (%) = [(viscosity after storage - viscosity before storage)/(viscosity before storage)] x 100     [Formula 1]

[Evaluation criteria]

A: The change rate of viscosity is less than ±5%
B: The change rate of viscosity is ±5% or greater and less than 10%
C: The change rate of viscosity is ±10% or greater and less than 15%
D: The change rate of viscosity is ±15% or greater

(Preliminary experiment 1)

<Preliminary investigation 1 on the ratio of anionic surfactant/amphoteric surfactant>

- A case of Mass ratio [(A) anionic surfactant/(B) amphoteric surfactant] = 0.3 to 4 -

According to Table 1-1 and Table 1-2, the hair cleansing agents of Test examples 1-1 to 1-9 were prepared by a common method to measure the pH and viscosity. The results are shown in Table 1-1 and Table 1-2.

[Table 1-1]

| Classification | Component name | Test example | | | | |
|---|---|---|---|---|---|---|
| | | 1-1 | 1-2 | 1-3 | 1-4 | 1-5 |
| Moisturizer | Sorbitol | 7.0 | 7.0 | 7.0 | 7.0 | 7.0 |
| | Isopentyldiol | 2.0 | 2.0 | 2.0 | 2.0 | 2.0 |
| Cationic polymer | Polyquaternium-10 | 0.5 | 0.5 | 0.5 | 0.5 | 0.5 |
| | Guar hydroxypropyltrimonium chloride | 0.2 | 0.2 | 0.2 | 0.2 | 0.2 |
| (A) Anionic surfactant | (A1) Sodium methyl cocoyl taurate | 1.5 | 2.1 | 3.0 | 3.6 | 4.5 |
| | (A2) Sodium lauroyl methylaminopropionate | 1.5 | 2.1 | 3.0 | 3.6 | 4.5 |
| (B) Amphoteric surfactant | Cocamidopropyl betaine | 12.0 | 10.8 | 9.0 | 7.8 | 6.0 |
| Nonionic surfactant | Cocamidomethyl MEA | 2.0 | 2.0 | 2.0 | 2.0 | 2.0 |
| Preservative | Phenoxy ethanol | Suitable amount | | | | |
| | Sodium benzoate | Suitable amount | | | | |
| Chelating agent | EDTA-2Na | Suitable amount | | | | |
| pH adjuster | Citric acid | Suitable amount | | | | |
| Solvent | Purified water | Remaining amount | | | | |
| Total (% by mass) | | 100 | 100 | 100 | 100 | 100 |
| Total content [(A)+(B)] | | 15.0 | 15.0 | 15.0 | 15.0 | 15.0 |
| Mass ratio [(A)/(B)] | | 0.3 | 0.4 | 0.7 | 0.9 | 1.5 |
| Mass ratio [(A1)/(A2)] | | 1.0 | 1.0 | 1.0 | 1.0 | 1.0 |
| pH | | 6.0 | 6.0 | 6.0 | 6.0 | 6.0 |
| Viscosity (25°C, mPa ▪ s)) | | 2,759 | 3,859 | 2,979 | 1,700 | 570 |

[Table 1-2]

| Classification | Component name | Test example | | | |
|---|---|---|---|---|---|
| | | 1-6 | 1-7 | 1-8 | 1-9 |
| Moisturizer | Sorbitol | 7.0 | 7.0 | 7.0 | 7.0 |
| | Isopentyldiol | 2.0 | 2.0 | 2.0 | 2.0 |
| Cationic polymer | Polyquaternium-10 | 0.5 | 0.5 | 0.5 | 0.5 |
| | Guar hydroxypropyltrimonium chloride | 0.2 | 0.2 | 0.2 | 0.2 |
| (A) Anionic surfactant | (A1) Sodium methyl cocoyl taurate | 5.1 | 6.0 | 6.0 | - |
| | (A2) Sodium lauroyl methylaminopropionate | 5.1 | 6.0 | - | 6.0 |
| (B) Amphoteric surfactant | Cocamidopropyl betaine | 4.8 | 3.0 | 9.0 | 9.0 |
| Nonionic surfactant | Cocamidomethyl MEA | 2.0 | 2.0 | 2.0 | 2.0 |
| Preservative | Phenoxy ethanol | Suitable amount | | | |
| | Sodium benzoate | Suitable amount | | | |
| Chelating agent | EDTA-2Na | Suitable amount | | | |
| pH adjuster | Citric acid | Suitable amount | | | |
| Solvent | Purified water | Remaining amount | | | |
| Total (% by mass) | | 100 | 100 | 100 | 100 |
| Total content [(A)+(B)] | | 15.0 | 15.0 | 15.0 | 15.0 |

(continued)

| Classification | Component name | Test example | | | |
|---|---|---|---|---|---|
| | | 1-6 | 1-7 | 1-8 | 1-9 |
| Mass ratio [(A)/(B)] | | 2.1 | 4.0 | 0.7 | 0.7 |
| Mass ratio [(A1)/(A2)] | | 1.0 | 1.0 | - | - |
| pH | | 6.0 | 6.0 | 6.0 | 6.0 |
| Viscosity (25°C, mPa ▪ s)) | | 340 | 230 | 370 | 870 |

From the results of Table 1-1 and Table 1-2, it was found by Test examples 1-1 to 1-7 that the viscosity changes by the mass ratio [(A) anionic surfactant/(B) amphoteric surfactant], and an effect of increasing the viscosity can be achieved within a range of the mass ratio [(A) anionic surfactant/(B) amphoteric surfactant] = 0.3 to 0.9.

Moreover, it was found by Test examples 1-3, 1-8 and 1-9 that, when the total contents of the surfactants were the same, the effect of increasing the viscosity is not exhibited with a single component of sodium methyl cocoyl taurate or sodium lauroyl methylaminopropionate, and the effect of increasing the viscosity is exhibited by using sodium methyl cocoyl taurate and sodium lauroyl methylaminopropionate together.

(Preliminary experiment 2-1)

<Preliminary investigation 2 on the ratio of anionic surfactant/amphoteric surfactant>

- A case of Mass ratio [(A) anionic surfactant/(B) amphoteric surfactant] = 0.7 -

According to Table 2-1 and Table 2-2, the hair cleansing agents of Test examples were prepared by a common method to measure the pH and viscosity. The results are shown in Table 2-1 and Table 2-2.

[Table 2-1]

| Classification | Component name | Test example | | | |
|---|---|---|---|---|---|
| | | 1-8 | 1-10 | 1-11 | 1-3 |
| Moisturizer | Sorbitol | 7.0 | 7.0 | 7.0 | 7.0 |
| | Isopentyldiol | 2.0 | 2.0 | 2.0 | 2.0 |
| Cationic polymer | Polyquaternium 10 | 0.5 | 0.5 | 0.5 | 0.5 |
| | Guar hydroxypropyltrimonium chloride | 0.2 | 0.2 | 0.2 | 0.2 |
| (A) Anionic surfactant | (A1) Sodium methyl cocoyl taurate | 6.0 | 5.0 | 4.0 | 3.0 |
| | (A2) Sodium lauroyl methylaminopropionate | - | 1.0 | 2.0 | 3.0 |
| (B) Amphoteric surfactant | Cocamidopropyl betaine | 9.0 | 9.0 | 9.0 | 9.0 |
| Nonionic surfactant | Cocamidomethyl MEA | 2.0 | 2.0 | 2.0 | 2.0 |
| Preservative | Phenoxy ethanol | Suitable amount | | | |
| | Sodium benzoate | Suitable amount | | | |
| Chelating agent | EDTA-2Na | Suitable amount | | | |
| pH adjuster | Citric acid | Suitable amount | | | |
| Solvent | Purified water | Remaining amount | | | |
| Total (% by mass) | | 100 | 100 | 100 | 100 |
| Total content [(A)+(B)] | | 15.0 | 15.0 | 15.0 | 15.0 |
| Mass ratio [(A)/(B)] | | 0.7 | 0.7 | 0.7 | 0.7 |
| Mass ratio [(A1)/(A2)] | | - | 5.0 | 2.0 | 1.0 |
| pH | | 6.0 | 6.0 | 6.0 | 6.0 |

(continued)

| Classification | Component name | Test example | | | |
|---|---|---|---|---|---|
| | | 1-8 | 1-10 | 1-11 | 1-3 |
| Viscosity (25°C, mPa ▪ s)) | | 370 | 1,279 | 2,669 | 2,979 |

[Table 2-2]

| Classification | Component name | Test example | | |
|---|---|---|---|---|
| | | 1-12 | 1-13 | 1-9 |
| Moisturizer | Sorbitol | 7.0 | 7.0 | 7.0 |
| | Isopentyldiol | 2.0 | 2.0 | 2.0 |
| Cationic polymer | Polyquaternium-10 | 0.5 | 0.5 | 0.5 |
| | Guar hydroxypropyltrimonium chloride | 0.2 | 0.2 | 0.2 |
| (A) Anionic surfactant | (A1) Sodium methyl cocoyl taurate | 2.0 | 1.0 | - |
| | (A2) Sodium lauroyl methylaminopropionate | 4.0 | 5.0 | 6.0 |
| (B) Amphoteric surfactant | Cocamidopropyl betaine | 9.0 | 9.0 | 9.0 |
| Nonionic surfactant | Cocamidomethyl MEA | 2.0 | 2.0 | 2.0 |
| Preservative | Phenoxy ethanol | Suitable amount | | |
| | Sodium benzoate | Suitable amount | | |
| Chelating agent | EDTA-2Na | Suitable amount | | |
| pH adjuster | Citric acid | Suitable amount | | |
| Solvent | Purified water | Remaining amount | | |
| Total (% by mass) | | 100 | 100 | 100 |
| Total content [(A)+(B)] | | 15.0 | 15.0 | 15.0 |
| Mass ratio [(A)/(B)] | | 0.7 | 0.7 | 0.7 |
| Mass ratio [(A1)/(A2)] | | 0.5 | 0.2 | - |
| pH | | 6.0 | 6.0 | 6.0 |
| Viscosity (25°C, mPa ▪ s)) | | 4,819 | 3,800 | 870 |

From the results of Table 2-1 and Table 2-2, it was found that variation in the viscosity occurs by the blending ratio of sodium methyl cocoyl taurate and sodium lauroyl methylaminopropionate.
Therefore, it is considered that a preferred range of exhibiting the effect increasing the viscosity is the mass ratio (sodium methyl cocoyl taurate/sodium lauroyl methylaminopropionate) = 0.2 to 2.

(Preliminary experiment 2-2)

<Preliminary investigation 2 on the ratio of anionic surfactant/amphoteric surfactant>

- A case of Mass ratio [(A) anionic surfactant/(B) amphoteric surfactant] = 0.3 -

According to Table 2-3 and Table 2-4, the hair cleansing agents of Test examples were prepared by a common method to measure the pH and viscosity. The results are shown in Table 2-3 and Table 2-4.

[Table 2-3]

| Classification | Component name | Test example | | | |
|---|---|---|---|---|---|
| | | 1-14 | 1-15 | 1-16 | 1-1 |
| Moisturizer | Sorbitol | 7.0 | 7.0 | 7.0 | 7.0 |
| | Isopentyldiol | 2.0 | 2.0 | 2.0 | 2.0 |
| Cationic polymer | Polyquaternium-10 | 0.5 | 0.5 | 0.5 | 0.5 |
| | Guar hydroxypropyltrimonium chloride | 0.2 | 0.2 | 0.2 | 0.2 |
| (A) Anionic surfactant | (A1) Sodium methyl cocoyl taurate | 3.0 | 2.5 | 2.0 | 1.5 |
| | (A2) Sodium lauroyl methylaminopropionate | - | 0.5 | 1.0 | 1.5 |
| (B) Amphoteric surfactant | Cocamidopropyl betaine | 12.0 | 12.0 | 12.0 | 12.0 |
| Nonionic surfactant | Cocamidomethyl MEA | 2.0 | 2.0 | 2.0 | 2.0 |
| Preservative | Phenoxy ethanol | Suitable amount | | | |
| | Sodium benzoate | Suitable amount | | | |
| Chelating agent | EDTA-2Na | Suitable amount | | | |
| pH adjuster | Citric acid | Suitable amount | | | |
| Solvent | Purified water | Remaining amount | | | |
| Total (% by mass) | | 100 | 100 | 100 | 100 |
| Total content [(A)+(B)] | | 15.0 | 15.0 | 15.0 | 15.0 |
| Mass ratio [(A)/(B)] | | 0.3 | 0.3 | 0.3 | 0.3 |
| Mass ratio [(A1)/(A2)] | | - | 5.0 | 2.0 | 1.0 |
| pH | | 6.0 | 6.0 | 6.0 | 6.0 |
| Viscosity (25°C, mPa ▪ s)) | | 1,169 | 1,289 | 2,529 | 2,759 |

[Table 2-4]

| Classification | Component name | Test example | | |
|---|---|---|---|---|
| | | 1-17 | 1-18 | 1-19 |
| Moisturizer | Sorbitol | 7.0 | 7.0 | 7.0 |
| | Isopentyldiol | 2.0 | 2.0 | 2.0 |
| Cationic polymer | Polyquaternium-10 | 0.5 | 0.5 | 0.5 |
| | Guar hydroxypropyltrimonium chloride | 0.2 | 0.2 | 0.2 |
| (A) Anionic surfactant | (A1) Sodium methyl cocoyl taurate | 1.0 | 0.5 | - |
| | (A2) Sodium lauroyl methylaminopropionate | 2.0 | 2.5 | 3.0 |
| (B) Amphoteric surfactant | Cocamidopropyl betaine | 12.0 | 12.0 | 12.0 |
| Nonionic surfactant | Cocamidomethyl MEA | 2.0 | 2.0 | 2.0 |
| Preservative | Phenoxy ethanol | Suitable amount | | |
| | Sodium benzoate | Suitable amount | | |
| Chelating agent | EDTA-2Na | Suitable amount | | |
| pH adjuster | Citric acid | Suitable amount | | |
| Solvent | Purified water | Remaining amount | | |
| Total (% by mass) | | 100 | 100 | 100 |
| Total content [(A)+(B)] | | 15.0 | 15.0 | 15.0 |

(continued)

| Classification | Component name | Test example | | |
|---|---|---|---|---|
| | | 1-17 | 1-18 | 1-19 |
| Mass ratio [(A)/(B)] | | 0.3 | 0.3 | 0.3 |
| Mass ratio [(A1)/(A2)] | | 0.5 | 0.2 | - |
| pH | | 6.0 | 6.0 | 6.0 |
| Viscosity (25°C, mPa ▪ s)) | | 3,419 | 3,149 | 1,080 |

From the results of Table 2-3 and Table 2-4, it was found that variation in the viscosity occurs by the blending ratio of sodium methyl cocoyl taurate and sodium lauroyl methylaminopropionate.

Therefore, similar to the above-described case of Mass ratio [(A) anionic surfactant/(B) amphoteric surfactant] = 0.7, it is considered that a preferred range of exhibiting the effect increasing the viscosity is the mass ratio (sodium methyl cocoyl taurate/sodium lauroyl methylaminopropionate) = 0.2 to 2.

(Preliminary experiment 2-3)

<Preliminary investigation 2 on the ratio of anionic surfactant/amphoteric surfactant>

- A case of Mass ratio [(A) anionic surfactant/(B) amphoteric surfactant] = 0.9 -

According to Table 2-5 and Table 2-6, the hair cleansing agents of Test examples were prepared by a common method to measure the pH and viscosity. The results are shown in Table 2-5 and Table 2-6.

[Table 2-5]

| Classification | Component name | Test example | | | |
|---|---|---|---|---|---|
| | | 1-20 | 1-21 | 1-22 | 1-4 |
| Moisturizer | Sorbitol | 7.0 | 7.0 | 7.0 | 7.0 |
| | Isopentyldiol | 2.0 | 2.0 | 2.0 | 2.0 |
| Cationic polymer | Polyquaternium-10 | 0.5 | 0.5 | 0.5 | 0.5 |
| | Guar hydroxypropyltrimonium chloride | 0.2 | 0.2 | 0.2 | 0.2 |
| (A) Anionic surfactant | (A1) Sodium methyl cocoyl taurate | 7.2 | 6.0 | 4.8 | 3.6 |
| | (A2) Sodium lauroyl methylaminopropionate | - | 1.2 | 2.4 | 3.6 |
| (B) Amphoteric surfactant | Cocamidopropyl betaine | 7.8 | 7.8 | 7.8 | 7.8 |
| Nonionic surfactant | Cocamidomethyl MEA | 2.0 | 2.0 | 2.0 | 2.0 |
| Preservative | Phenoxy ethanol | Suitable amount | | | |
| | Sodium benzoate | Suitable amount | | | |
| Chelating agent | EDTA-2Na | Suitable amount | | | |
| pH adjuster | Citric acid | Suitable amount | | | |
| Solvent | Purified water | Remaining amount | | | |
| Total (% by mass) | | 100 | 100 | 100 | 100 |
| Total content [(A)+(B)] | | 15.0 | 15.0 | 15.0 | 15.0 |
| Mass ratio [(A)/(B)] | | 0.9 | 0.9 | 0.9 | 0.9 |
| Mass ratio [(A1)/(A2)] | | - | 5.0 | 2.0 | 1.0 |
| pH | | 6.0 | 6.0 | 6.0 | 6.0 |
| Viscosity (25°C, mPa ▪ s)) | | 440 | 660 | 1,560 | 1,700 |

[Table 2-6]

| Classification | Component name | Test example | | |
|---|---|---|---|---|
| | | 1-23 | 1-24 | 1-25 |
| Moisturizer | Sorbitol | 7.0 | 7.0 | 7.0 |
| | Isopentyldiol | 2.0 | 2.0 | 2.0 |
| Cationic polymer | Polyquaternium-10 | 0.5 | 0.5 | 0.5 |
| | Guar hydroxypropyltrimonium chloride | 0.2 | 0.2 | 0.2 |
| (A) Anionic surfactant | (A1) Sodium methyl cocoyl taurate | 2.4 | 1.2 | - |
| | (A2) Sodium lauroyl methylaminopropionate | 4.8 | 6.0 | 7.2 |
| (B) Amphoteric surfactant | Cocamidopropyl betaine | 7.8 | 7.8 | 7.8 |
| Nonionic surfactant | Cocamidomethyl MEA | 2.0 | 2.0 | 2.0 |
| Preservative | Phenoxy ethanol | Suitable amount | | |
| | Sodium benzoate | Suitable amount | | |
| Chelating agent | EDTA-2Na | Suitable amount | | |
| pH adjuster | Citric acid | Suitable amount | | |
| Solvent | Purified water | Remaining amount | | |
| Total (% by mass) | | 100 | 100 | 100 |
| Total content [(A)+(B)] | | 15.0 | 15.0 | 15.0 |
| Mass ratio [(A)/(B)] | | 0.9 | 0.9 | 0.9 |
| Mass ratio [(A1)/(A2)] | | 0.5 | 0.2 | - |
| pH | | 6.0 | 6.0 | 6.0 |
| Viscosity (25°C, mPa ▪ s)) | | 3,639 | 4,809 | 1,208 |

From the results of Table 2-5 and Table 2-6, it was found that variation in the viscosity occurs by the blending ratio of sodium methyl cocoyl taurate and sodium lauroyl methylaminopropionate.

Therefore, similar to the above-described case of Mass ratio [(A) anionic surfactant/(B) amphoteric surfactant] = 0.7, it is considered that a preferred range of exhibiting the effect increasing the viscosity is the mass ratio (sodium methyl cocoyl taurate/sodium lauroyl methylaminopropionate) = 0.2 to 2.

(Preliminary experiment 3)

<Preliminary investigation on the total content of (A) anionic surfactant and (B) amphoteric surfactant >

According to Table 3, the hair cleansing agents of Test examples 2-1 to 2-4 were prepared by a common method to evaluate foaming property and measure the pH and viscosity. The results are shown in Table 3.

[Table 3]

| Classification | Component name | Test example | | | | |
|---|---|---|---|---|---|---|
| | | 2-1 | 2-2 | 1-3 | 2-3 | 2-4 |
| Moisturizer | Sorbitol | 7.0 | 7.0 | 7.0 | 7.0 | 7.0 |
| | Isopentyldiol | 2.0 | 2.0 | 2.0 | 2.0 | 2.0 |
| Cationic polymer | Polyquaternium-10 | 0.5 | 0.5 | 0.5 | 0.5 | 0.5 |
| | Guar hydroxypropyltrimonium chloride | 0.3 | 0.3 | 0.3 | 0.3 | 0.3 |
| (A) Anionic surfactant | (A1) Sodium methyl cocoyl taurate | 1.0 | 2.0 | 3.0 | 4.0 | 5.0 |
| | (A2) Sodium lauroyl methylaminopropionate | 1.0 | 2.0 | 3.0 | 4.0 | 5.0 |

(continued)

| Classification | Component name | Test example | | | | |
|---|---|---|---|---|---|---|
| | | 2-1 | 2-2 | 1-3 | 2-3 | 2-4 |
| (B) Amphoteric surfactant | Cocamidopropyl betaine | 3.0 | 6.0 | 9.0 | 12.0 | 15.0 |
| Nonionic surfactant | Cocamidomethyl MEA | 2.0 | 2.0 | 2.0 | 2.0 | 2.0 |
| Preservative | Phenoxy ethanol | Suitable amount | | | | |
| | Sodium benzoate | Suitable amount | | | | |
| Chelating agent | EDTA-2Na | Suitable amount | | | | |
| pH adjuster | Citric acid | Suitable amount | | | | |
| Solvent | Purified water | Remaining amount | | | | |
| Total (% by mass) | | 100 | 100 | 100 | 100 | 100 |
| Total content [(A)+(B)] | | 5.0 | 10.0 | 15.0 | 20.0 | 25.0 |
| Mass ratio [(A)/(B)] | | 0.7 | 0.7 | 0.7 | 0.7 | 0.7 |
| Mass ratio [(A1)/(A2)] | | 1.0 | 1.0 | 1.0 | 1.0 | 1.0 |
| Foaming property | | C | A | A | A | A |
| pH | | 6.0 | 6.0 | 6.0 | 6.0 | 6.0 |
| Viscosity (25°C, mPa ∎ s)) | | 1,320 | 3,799 | 2,979 | 2,949 | 2,870 |

From the results of Table 3, since the viscosity and foaming property are insufficient when the total content is 5% by mass in Test example 2-1, it is considered that the total content of (A) anionic surfactant and (B) amphoteric surfactant is preferably 10 to 25% by mass in terms of foaming property and the effect of increasing viscosity.

(Preliminary experiment 4)

<Preliminary investigation on the content of PEG-120 methyl glucose dioleate>

According to Table 4-1 and Table 4-2, the hair cleansing agents of Test examples were prepared by a common method to measure the pH and viscosity. The results are shown in Table 4-1 and Table 4-2.

[Table 4-1]

| Classification | Component name | Test example | | | | | |
|---|---|---|---|---|---|---|---|
| | | 1-3 | 3-1 | 3-2 | 3-3 | 3-4 | 1-2 |
| Moisturizer | Sorbitol | 7.0 | 7.0 | 7.0 | 7.0 | 7.0 | 7.0 |
| | Isopentyldiol | 2.0 | 2.0 | 2.0 | 2.0 | 2.0 | 2.0 |
| Cationic polymer | Polyquaternium-10 | 0.5 | 0.5 | 0.5 | 0.5 | 0.5 | 0.5 |
| | Guar hydroxypropyltrimonium chloride | 0.3 | 0.3 | 0.3 | 0.3 | 0.3 | 0.2 |
| (A) Anionic surfactant | (A1) Sodium methyl cocoyl taurate | 3.0 | 3.0 | 3.0 | 3.0 | 3.0 | 2.1 |
| | (A2) Sodium lauroyl methylaminopropionate | 3.0 | 3.0 | 3.0 | 3.0 | 3.0 | 2.1 |
| (B) Amphoteric surfactant | Cocamidopropyl betaine | 9.0 | 9.0 | 9.0 | 9.0 | 9.0 | 10.8 |
| Viscosity modifier | PEG-120 methyl glucose dioleate | - | 1.0 | 2.0 | 3.0 | 4.0 | - |

(continued)

| Classification | Component name | Test example | | | | | |
|---|---|---|---|---|---|---|---|
| | | 1-3 | 3-1 | 3-2 | 3-3 | 3-4 | 1-2 |
| Nonionic surfac-tant | Cocamidomethyl MEA | 2.0 | 2.0 | 2.0 | 2.0 | 2.0 | 2.0 |
| Preservative | Phenoxy ethanol | Suitable amount | | | | | |
| | Sodium benzoate | Suitable amount | | | | | |
| Chelating agent | EDTA-2Na | Suitable amount | | | | | |
| pH adjuster | Citric acid | Suitable amount | | | | | |
| Solvent | Purified water | Remaining amount | | | | | |
| Total (% by mass) | | 100 | 100 | 100 | 100 | 100 | 100 |
| Total content [(A)+(B)] | | 15.0 | 15.0 | 15.0 | 15.0 | 15.0 | 15.0 |
| Mass ratio [(A)/(B)] | | 0.7 | 0.7 | 0.7 | 0.7 | 0.7 | 0.4 |
| Mass ratio [(A1)/(A2)] | | 1.0 | 1.0 | 1.0 | 1.0 | 1.0 | 1.0 |
| pH | | 6.0 | 6.0 | 6.0 | 6.0 | 6.0 | 6.0 |
| Viscosity (25°C, mPa ▪ s)) | | 2,979 | 6,400 | 15,200 | 27,000 | 35,500 | 3,859 |

[Table 4-2]

| Classification | Component name | Test example | | | | |
|---|---|---|---|---|---|---|
| | | 3-5 | 1-5 | 3-6 | 1-6 | 3-7 |
| Moisturizer | Sorbitol | 7.0 | 7.0 | 7.0 | 7.0 | 7.0 |
| | Isopentyldiol | 2.0 | 2.0 | 2.0 | 2.0 | 2.0 |
| Cationic polymer | Polyquaternium-10 | 0.5 | 0.5 | 0.5 | 0.5 | 0.5 |
| | Guar hydroxypropyltrimonium chloride | 0.3 | 0.2 | 0.3 | 0.2 | 0.3 |
| (A) Anionic surfactant | (A1) Sodium methyl cocoyl taurate | 2.1 | 4.5 | 4.5 | 5.1 | 5.1 |
| | (A2) Sodium lauroyl methylaminopropionate | 2.1 | 4.5 | 4.5 | 5.1 | 5.1 |
| (B) Amphoteric surfactant | Cocamidopropyl betaine | 10.8 | 6.0 | 6.0 | 4.8 | 4.8 |
| Viscosity modifier | PEG-120 methyl glucose dioleate | 1.0 | - | 1.0 | - | 1.0 |
| Nonionic surfactant | Cocamidomethyl MEA | 2.0 | 2.0 | 2.0 | 2.0 | 2.0 |
| Preservative | Phenoxy ethanol | Suitable amount | | | | |
| | Sodium benzoate | Suitable amount | | | | |
| Chelating agent | EDTA-2Na | Suitable amount | | | | |
| pH adjuster | Citric acid | Suitable amount | | | | |
| Solvent | Purified water | Remaining amount | | | | |
| Total (% by mass) | | 100 | 100 | 100 | 100 | 100 |
| Total content [(A)+(B)] | | 15.0 | 15.0 | 15.0 | 15.0 | 15.0 |
| Mass ratio [(A)/(B)] | | 0.4 | 1.5 | 1.5 | 2.1 | 2.1 |
| Mass ratio [(A1)/(A2)] | | 1.0 | 1.0 | 1.0 | 1.0 | 1.0 |
| pH | | 6.0 | 6.0 | 6.0 | 6.0 | 6.0 |
| Viscosity (25°C, mPa ▪ s)) | | 14,500 | 570 | 280 | 340 | 170 |

From the results of Table 4-1 and Table 4-2, it was observed in Test examples 1-3 to 3-4 that the effect of increasing the viscosity is exhibited by incorporating PEG-120 methyl glucose dioleate.

On the other hand, by comparing Test example 1-2 and Test example 3-5, Test example 1-5 and Test example 3-6, and Test example 1-6 and Test example 3-7, it was found that even the blending amount of PEG-120 methyl glucose dioleate is the same, the viscosity does not increase in the levels of the mass ratio [(A) anionic surfactant/(B) amphoteric surfactant] = 1.5, 2.1 (Test example 1-5, Test example 1-6).

Therefore, since the effect of increasing the viscosity by incorporating PEG-120 methyl glucose dioleate is not observed when the anionic surfactant is incorporated in a larger amount than the amphoteric surfactant, it is considered that, similar to the preliminary investigation in Test examples 1-1 to 1-7, the viscosity increases specifically in the mass ratio [(A) anionic surfactant/(B) amphoteric surfactant] = 0.3 to 0.9.

(Preliminary experiment 5)

<Preliminary investigation on the blending amount of acrylates copolymer>

According to Table 5, the hair cleansing agents of Test examples were prepared by a common method to evaluate usability and measure the pH and viscosity. The results are shown in Table 5.

[Table 5]

| Classification | Component name | Test example | | | | |
|---|---|---|---|---|---|---|
| | | 1-3 | 4-1 | 4-2 | 4-3 | 4-4 |
| Moisturizer | Sorbitol | 7.0 | 7.0 | 7.0 | 7.0 | 7.0 |
| | Isopentyldiol | 2.0 | 2.0 | 2.0 | 2.0 | 2.0 |
| Cationic polymer | Polyquaternium-10 | 0.5 | 0.5 | 0.5 | 0.5 | 0.5 |
| | Guar hydroxypropyltrimonium chloride | 0.3 | 0.3 | 0.3 | 0.3 | 0.3 |
| (A) Anionic surfactant | (A1) Sodium methyl cocoyl taurate | 3.0 | 3.0 | 3.0 | 3.0 | 3.0 |
| | (A2) Sodium lauroyl methylaminopropionate | 3.0 | 3.0 | 3.0 | 3.0 | 3.0 |
| (B) Amphoteric surfactant | Cocamidopropyl betaine | 9.0 | 9.0 | 9.0 | 9.0 | 9.0 |
| Viscosity modifier | Acrylates copolymer | - | 0.3 | 0.9 | 1.5 | 2.1 |
| Neutralizer | Potassium hydroxide | - | 0.05 | 0.15 | 0.25 | 0.35 |
| Nonionic surfactant | Cocamidomethyl MEA | 2.0 | 2.0 | 2.0 | 2.0 | 2.0 |
| Preservative | Phenoxy ethanol | Suitable amount | | | | |
| | Sodium benzoate | Suitable amount | | | | |
| Chelating agent | EDTA-2Na | Suitable amount | | | | |
| pH adjuster | Citric acid | Suitable amount | | | | |
| Solvent | Purified water | Remaining amount | | | | |
| Total (% by mass) | | 100 | 100 | 100 | 100 | 100 |
| Total content [(A)+(B)] | | 15.0 | 15.0 | 15.0 | 15.0 | 15.0 |
| Mass ratio [(A)/(B)] | | 0.7 | 0.7 | 0.7 | 0.7 | 0.7 |
| Mass ratio [(A1)/(A2)] | | 1.0 | 1.0 | 1.0 | 1.0 | 1.0 |
| pH | | 6.0 | 6.0 | 6.0 | 6.0 | 6.0 |
| Viscosity (25°C, mPa ▪ s)) | | 2,979 | 3,109 | 3,459 | 4,459 | 12,600 |
| Usability | | A | A | A | B | C |

From the results of Table 5, the viscosity tended to increase depending on the blending amount of acrylates copolymer; however, from about 1.5% by mass, smoothness of combing hair with fingers at use deteriorated and squeakiness was felt.

Therefore, in the hair cleansing agent, the blending amount of acrylates copolymer is preferably 0.1% by mass to 1.2% by mass, and more preferably 0.3% by mass to 0.9% by mass.

(Test examples 5-1 to 5-8)

<Preparation of the hair cleansing agent>

According to Table 6-1 and Table 6-2, the hair cleansing agents of Test examples 5-1 to 5-8 were prepared by a common method to evaluate usability, foaming property and storage stability at high temperature, and measure the pH and viscosity. The results are shown in Table 6-1 and Table 6-2.

[Table 6-1]

| Classification | Component name | Test example | | | | |
|---|---|---|---|---|---|---|
| | | 5-1 | 5-2 | 5-3 | 5-4 | 5-5 |
| Moisturizer | Sorbitol | 7.0 | 7.0 | 7.0 | 7.0 | 7.0 |
| | Isopentyldiol | 2.0 | 2.0 | 2.0 | 2.0 | 2.0 |
| Cationic polymer | Polyquaternium-10 | 0.5 | 0.5 | 0.5 | 0.5 | 0.5 |
| | Guar hydroxypropyltrimonium chloride | 0.3 | 0.3 | 0.3 | 0.3 | 0.3 |
| (A) Anionic surfactant | (A1) Sodium methyl cocoyl taurate | 3.0 | 3.0 | 3.0 | 3.0 | 3.0 |
| | (A2) Sodium lauroyl methylaminopropionate | 3.0 | 3.0 | 3.0 | 3.0 | 3.0 |
| (B) Amphoteric surfactant | Cocamidopropyl betaine | 9.0 | 9.0 | 9.0 | 9.0 | 9.0 |
| Viscosity modifier | PEG-120 methyl glucose dioleate | 1.0 | - | 1.0 | 2.0 | - |
| | Acrylates copolymer | - | 0.3 | 0.3 | - | 0.9 |
| Neutralizer | Potassium hydroxide | - | 0.05 | 0.05 | - | 0.15 |
| Nonionic surfactant | Cocamidomethyl MEA | 2.0 | 2.0 | 2.0 | 2.0 | 2.0 |
| Preservative | Phenoxy ethanol | Suitable amount | | | | |
| | Sodium benzoate | Suitable amount | | | | |
| Chelating agent | EDTA-2Na | Suitable amount | | | | |
| pH adjuster | Citric acid | Suitable amount | | | | |
| Solvent | Purified water | Remaining amount | | | | |
| Total (% by mass) | | 100 | 100 | 100 | 100 | 100 |
| Total content [(A)+(B)] | | 15.0 | 15.0 | 15.0 | 15.0 | 15.0 |
| Mass ratio [(A)/(B)] | | 0.7 | 0.7 | 0.7 | 0.7 | 0.7 |
| Mass ratio [(A1)/(A2)] | | 1.0 | 1.0 | 1.0 | 1.0 | 1.0 |
| pH | | 6.0 | 6.0 | 6.0 | 6.0 | 6.0 |
| Viscosity (25°C, mPa ▪ s)) | | 6,400 | 3,109 | 10,500 | 15,200 | 3,459 |
| Storage stability at high temperature | | C | B | A | C | B |
| Foaming property | | A | B | A | A | B |
| Usability | | A | A | A | A | A |

[Table 6-2]

| Classification | Component name | Test example | | |
|---|---|---|---|---|
| | | 5-6 | 5-7 | 5-8 |
| Moisturizer | Sorbitol | 7.0 | 7.0 | 7.0 |
| | Isopentyldiol | 2.0 | 2.0 | 2.0 |

(continued)

| Classification | Component name | Test example | | |
|---|---|---|---|---|
| | | 5-6 | 5-7 | 5-8 |
| Cationic polymer | Polyquaternium-10 | 0.5 | 0.5 | 0.5 |
| | Guar hydroxypropyltrimonium chloride | 0.3 | 0.3 | 0.3 |
| (A) Anionic surfactant | (A1) Sodium methyl cocoyl taurate | 3.0 | 3.0 | 3.0 |
| | (A2) Sodium lauroyl methylaminopropionate | 3.0 | 3.0 | 3.0 |
| (B) Amphoteric surfactant | Cocamidopropyl betaine | 9.0 | 9.0 | 9.0 |
| Viscosity modifier | PEG-120 methyl glucose dioleate | 2.0 | 3.0 | 0.5 |
| | Acrylates copolymer | 0.9 | 0.3 | 0.3 |
| Neutralizer | Potassium hydroxide | 0.15 | 0.05 | 0.05 |
| Nonionic surfactant | Cocamidomethyl MEA | 2.0 | 2.0 | 2.0 |
| Preservative | Phenoxy ethanol | Suitable amount | | |
| | Sodium benzoate | Suitable amount | | |
| Chelating agent | EDTA-2Na | Suitable amount | | |
| pH adjuster | Citric acid | Suitable amount | | |
| Solvent | Purified water | Remaining amount | | |
| Total (% by mass) | | 100 | 100 | 100 |
| Total content [(A)+(B)] | | 15.0 | 15.0 | 15.0 |
| Mass ratio [(A)/(B)] | | 0.7 | 0.7 | 0.7 |
| Mass ratio [(A1)/(A2)] | | 1.0 | 1.0 | 1.0 |
| pH | | 6.0 | 6.0 | 6.0 |
| Viscosity (25°C, mPa ▪ s)) | | 22,700 | 27,000 | 4,690 |
| Storage stability at high temperature | | A | A | B |
| Foaming property | | A | A | A |
| Usability | | A | A | A |

From the results of Table 6-1 and Table 6-2, Test example 5-3 and Test examples 5-6 to 5-7 all satisfy the requirements of "comprising (A) acyl methyl taurate and acyl methyl alaninate; (B) at least one selected from alkylamidopropyl betaine and imidazolinium betaine; (C) 0.5% by mass to 3% by mass of polyoxyethylene methyl glucoside dioleate; and (D) at least one selected from an acrylic polymer and a reaction product of adipic acid and acetic anhydride and corn starch; and the total content of the component (A) and the component (B) is 10% by mass to 25% by mass, a mass ratio of the component (A) and the component (B) [(A)/(B)] is 0.3 to 0.9, and viscosity at 25C° is 6,000 mPa·s or greater", and the gel hair cleansing agent that can achieve a highly viscous gel, is excellent in foaming property and usability, and has a good storage stability at high temperature can be acquired.

On the other hand, since both Test example 5-1 and Test example 5-4 do not comprise (D) acrylic polymer, storage stability at high temperature is poor.

Test example 5-2 and Test example 5-5 do not comprise (C) polyoxyethylene methyl glucoside dioleate; therefore, the viscosity is low, and a highly viscous gel cannot be achieved.

Test example 5-8 does not satisfy the lower limit value of the content of (C) polyoxyethylene methyl glucoside dioleate, 1.0% by mass or greater; therefore, the viscosity is high, and a highly viscous gel cannot be achieved.

Therefore, to achieve a highly viscous gel and storage stability at high temperature, 0.5% by mass to 3% by mass of (C) polyoxyethylene methyl glucoside dioleate, and (D) acrylic polymer need to be incorporated together.

(Test examples 6-1 to 6-11)

<Preparation of the hair cleansing agent>

According to Table 7-1 and Table 7-2, the hair cleansing agents of Test examples 6-1 to 6-11 were prepared by a common method to evaluate usability, foaming property and storage stability at high temperature, and measure the pH and viscosity. The results are shown in Table 7-1 and Table 7-2.

[Table 7-1]

| Classification | Component name | Test example | | | | | |
|---|---|---|---|---|---|---|---|
| | | 6-1 | 6-2 | 6-3 | 6-4 | 6-5 | 6-6 |
| Moisturizer | Sorbitol | 15.0 | 15.0 | 15.0 | 15.0 | 15.0 | 15.0 |
| | Isopentyldiol | 2.0 | 2.0 | 2.0 | 2.0 | 2.0 | 2.0 |
| Cationic polymer | Polyquaternium-10 | 0.6 | 0.6 | 0.6 | 0.6 | 0.6 | 0.6 |
| | Guar hydroxypropyltrimonium chloride | 0.3 | 0.3 | 0.3 | 0.3 | 0.3 | 0.3 |
| | Polyquaternium-7 | 0.4 | 0.4 | 0.4 | 0.4 | 0.4 | 0.4 |
| (A) Anionic surfactant | (A1) Sodium methyl cocoyl taurate | 3.0 | 3.0 | 3.0 | 1.0 | 2.0 | 5.0 |
| | (A2) Sodium lauroyl methylaminopropionate | 3.0 | 3.0 | 3.0 | 1.0 | 2.0 | 5.0 |
| (B) Amphoteric surfactant | Cocamidopropyl betaine | 9.0 | 9.0 | 9.0 | 3.0 | 6.0 | 15.0 |
| | Sodium lauroamphoacetate | - | 3.0 | 3.0 | - | - | - |
| Viscosity modifier | PEG-120 methyl glucose dioleate | 2.0 | 2.0 | 2.0 | 2.0 | 2.0 | 2.0 |
| | Acrylates copolymer | 0.3 | 0.3 | - | 0.3 | 0.3 | 0.3 |
| | Starch acetate/adipate | - | - | 0.3 | - | - | - |
| Neutralizer | Potassium hydroxide | 0.05 | 0.05 | 0.05 | 0.05 | 0.05 | 0.05 |
| Nonionic surfactant | Cocamidomethyl MEA | 2.0 | 2.0 | 2.0 | 2.0 | 2.0 | 2.0 |
| Silicone | Dimethicone | 0.5 | 0.5 | 0.5 | 0.5 | 0.5 | 0.5 |
| Preservative | Phenoxy ethanol | Suitable amount | | | | | |
| | Sodium benzoate | Suitable amount | | | | | |
| Chelating agent | EDTA-2Na | Suitable amount | | | | | |
| pH adjuster | Citric acid | Suitable amount | | | | | |
| Solvent | Purified water | Remaining amount | | | | | |
| Total (% by mass) | | 100 | 100 | 100 | 100 | 100 | 100 |
| Total content [(A)+(B)] | | 15.0 | 18.0 | 18.0 | 5.0 | 10.0 | 25.0 |
| Mass ratio [(A)/(B)] | | 0.7 | 0.7 | 0.7 | 0.7 | 0.7 | 0.7 |
| Mass ratio [(A1)/(A2)] | | 1.0 | 1.0 | 1.0 | 1.0 | 1.0 | 1.0 |
| pH | | 6.0 | 6.0 | 6.0 | 6.0 | 6.0 | 6.0 |
| Viscosity (25°C. mPa ▪ s)) | | 11,800 | 10,200 | 12,500 | 1,520 | 6,790 | 13,870 |
| Storage stability at high temperature | | A | A | A | A | A | A |
| Foaming property | | A | A | A | C | A | A |
| Usability | | A | A | A | A | A | A |

[Table 7-2]

| Classification | Component name | Test example | | | | |
|---|---|---|---|---|---|---|
| | | 6-7 | 6-8 | 6-9 | 6-10 | 6-11 |
| Moisturizer | Sorbitol | 15.0 | 15.0 | 15.0 | 15.0 | 15.0 |
| | Isopentyldiol | 2.0 | 2.0 | 2.0 | 2.0 | 2.0 |
| Cationic polymer | Polyquaternium-10 | 0.6 | 0.6 | 0.6 | 0.6 | 0.6 |
| | Guar hydroxypropyltrimonium chloride | 0.3 | 0.3 | 0.3 | 0.3 | 0.3 |
| | Polyquaternium-7 | 0.4 | 0.4 | 0.4 | 0.4 | 0.4 |
| (A) Anionic surfactant | (A1) Sodium methyl cocoyl taurate | 1.5 | 3.6 | 4.5 | 1.0 | 4.0 |
| | (A2) Sodium lauroyl methylaminopropionate | 1.5 | 3.6 | 4.5 | 5.0 | 2.0 |
| (B) Amphoteric surfactant | Cocamidopropyl betaine | 12.0 | 7.8 | 6.0 | 9.0 | 9.0 |
| | Sodium lauroamphoacetate | - | - | - | - | - |
| Viscosity modifier | PEG-120 methyl glucose dioleate | 2.0 | 2.0 | 2.0 | 2.0 | 2.0 |
| | Acrylates copolymer | 0.3 | 0.3 | - | 0.3 | 0.3 |
| | Starch acetate/adipate | - | - | 0.3 | - | - |
| Neutralizer | Potassium hydroxide | 0.05 | 0.05 | 0.05 | 0.05 | 0.05 |
| Nonionic surfactant | Cocamidomethyl MEA | 2.0 | 2.0 | 2.0 | 2.0 | 2.0 |
| Silicone | Dimethicone | 0.5 | 0.5 | 0.5 | 0.5 | 0.5 |
| Preservative | Phenoxy ethanol | Suitable amount | | | | |
| | Sodium benzoate | Suitable amount | | | | |
| Chelating agent | EDTA-2Na | Suitable amount | | | | |
| pH adjuster | Citric acid | Suitable amount | | | | |
| Solvent | Purified water | Remaining amount | | | | |
| Total (% by mass) | | 100 | 100 | 100 | 100 | 100 |
| Total content [(A)+(B)] | | 15.0 | 15.0 | 15.0 | 15.0 | 15.0 |
| Mass ratio [(A)/(B)] | | 0.3 | 0.9 | 1.5 | 0.7 | 0.7 |
| Mass ratio [(A1)/(A2)] | | 1.0 | 1.0 | 1.0 | 0.2 | 2.0 |
| pH | | 6.0 | 6.0 | 6.0 | 6.0 | 6.0 |
| Viscosity (25°C, mPa ∎ s)) | | 11,700 | 8,700 | 4,700 | 9,620 | 7,560 |
| Storage stability at high temperature | | A | A | A | A | A |
| Foaming property | | A | A | A | A | A |
| Usability | | A | A | A | A | A |

From the results of Table 7-1 and Table 7-2, it was found that Test examples 6-1 to 6-3, Test examples 6-5 to 6-8, and Test examples 6-10 to 6-11 all satisfy the requirements of "comprising (A) acyl methyl taurate and acyl methyl alaninate; (B) at least one selected from alkylamidopropyl betaine and imidazolinium betaine; (C) 0.5% by mass to 3% by mass of polyoxyethylene methyl glucoside dioleate; and (D) at least one selected from an acrylic polymer and a reaction product of adipic acid and acetic anhydride and corn starch; and the total content of the component (A) and the component (B) is 10% by mass to 25% by mass, a mass ratio of the component (A) and the component (B) [(A)/(B)] is 0.3 to 0.9, and viscosity at 25C° is 6,000 mPa·s or greater", and the gel hair cleansing agent that can achieve a highly-viscous gel, is excellent in foaming property and usability, and has a good storage stability at high temperature can be acquired.

In the Test example 6-4, the total content of the component (A) and the component (B) is 5.0% by mass, and the lower limit value of the total content of the component (A) and the component (B), 10% by mass or greater, is not satisfied; therefore, the viscosity is low, a highly viscous gel cannot be achieved, and foaming property is poor.

In the Test example 6-9, the mass ratio [(A)/(B)] of the component (A) and the component (B) is 1.5, and the upper limit value of the mass ratio [(A)/(B)], 0.9 or lower, is not satisfied, therefore, the viscosity is low, and a highly viscous gel cannot be achieved.

Therefore, to achieve a highly viscous gel and storage stability at high temperature, it is necessary to incorporate (C) polyoxyethylene methyl glucoside dioleate, and (D) at least one selected from an acrylic polymer and a reaction product of adipic acid and acetic anhydride and corn starch together.

**Claims**

1. A gel hair cleansing agent comprising:

   (A) acyl methyl taurate and acyl methyl alaninate;
   (B) at least one selected from alkylamidopropyl betaine and imidazolinium betaine;
   (C) 0.5% by mass to 3% by mass of polyoxyethylene methyl glucoside dioleate; and
   (D) at least one selected from an acrylic polymer and a reaction product of adipic acid and acetic anhydride and corn starch,

   wherein the total content of the component (A) and the component (B) is 10% by mass to 25% by mass, a mass ratio of the component (A) and the component (B) [(A)/(B)] is 0.3 to 0.9, and viscosity at 25C° is 6,000 mPa·s or greater.

2. The gel hair cleansing agent according to claim 1, wherein the content of the component (A) is 1 % by mass to 15 % by mass.

3. The gel hair cleansing agent according to claim 1 or 2, wherein a mass ratio of acyl methyl taurate (A1) and acyl methyl alaninate (A2) [(A1)/(A2)] is 0.2 to 2.

4. The gel hair cleansing agent according to any one of claims 1 to 3, wherein the component (B) comprises at least one selected from cocamidopropyl betaine and sodium lauroamphoacetate.

5. The gel hair cleansing agent according to any one of claims 1 to 4, wherein the component (C) comprises PEG-120 methyl glucose dioleate.

6. The gel hair cleansing agent according to any one of claims 1 to 5, wherein the component (D) comprises at least one selected from acrylates copolymer and starch acetate/adipate.

7. The gel hair cleansing agent according to any one of claims 1 to 6, wherein the content of the component (D) is 0.1% by mass to 1.2% by mass.

8. The gel hair cleansing agent according to any one of claims 1 to 7, further comprising (E) cationic polymer.

9. The gel hair cleansing agent according to any one of claims 1 to 8, wherein the pH is 7 or lower.

10. The gel hair cleansing agent according to any one of claims 1 to 9 that is used in shampoo or an all-in-one shampoo.

Europäisches
Patentamt
European
Patent Office
Office européen
des brevets

# EUROPEAN SEARCH REPORT

**Application Number**

EP 25 22 4532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE GNPD [Online] "MINTEL"; 18 October 2022 (2022-10-18), "Amino-Acids Shampoo Secret Garden", XP093397880, Database accession no. 9993414 * the whole document * ----- | 1-10 | INV. A61K8/04 A61K8/44 A61K8/46 A61K8/60 A61Q5/02 A61K8/49 A61K8/81 |
| Y | Anonymous: "Soft Cleanse Scalp & Fiber Conditioning Shampoo", , 26 September 2021 (2021-09-26), pages 1-2, XP093286806, Retrieved from the Internet: URL:https://www.lubrizol.com/-/media/Lubrizol/Personal-Care/Documents/Formulations/Hair-Care/SH0199AP.pdf [retrieved on 2026-05-18] * the whole document * ----- | 1-10 | ADD. A61K8/86 |
| Y | CN 108 619 027 A (SHANGHAI NEW COGI COSMETIC CO LTD) 9 October 2018 (2018-10-09) * the whole document * * page 2, line 1 - line 7 * ----- | 1-10 | **TECHNICAL FIELDS SEARCHED (IPC)** A61K A61Q |
| Y | CN 110 327 238 A (SUNRISE HEALTH TECH GUANGZHOU CO LTD) 15 October 2019 (2019-10-15) * example 3 * ----- | 1-10 | |

The present search report has been drawn up for all claims

| Place of search | Date of completion of the search | Examiner |
|---|---|---|
| Munich | 21 May 2026 | Wäntig, Albrecht |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
........................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 25 22 4532

This annex lists the patent family members relating to the patent documents cited in the above-mentioned European search report.
The members are as contained in the European Patent Office EDP file on
The European Patent Office is in no way liable for these particulars which are merely given for the purpose of information.

21-05-2026

| Patent document cited in search report | Publication date | Patent family member(s) | Publication date |
|---|---|---|---|
| CN 108619027 A | 09-10-2018 | NONE | |
| CN 110327238 A | 15-10-2019 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6181309 B

**Non-patent literature cited in the description**

- ACRYLATES COPOLYMER. International Cosmetic Ingredient Dictionary and Handbook. CTFA, 2012, vol. 1, 60-61